# EUROPÄISCHE PATENTSCHRIFT

(11) **EP 1 986 005 B1**
(45) Veröffentlichungstag und Bekanntmachung des Hinweises auf die Patenterteilung: **13.01.2010**
(21) Anmeldenummer: 07008468.6
(22) Anmeldetag: 26.04.2007
(51) Int. Cl.: G01N 33/36

(54) **Frequenzabhängige Fehlstellenermittlung in einem Garn oder Garnvorgänger**
Frequency-dependent flaw detection in a yarn or a yarn forerunner
Signal de positionnement erroné en fonction de la fréquence dans un fil ou un antécédent de fil

(43) Veröffentlichungstag der Anmeldung: 29.10.2008
(73) Patentinhaber: Gebrüder Loepfe AG, CH-8623 Wetzikon (CH)
(72) Erfinder: Mauron, Pascal, 8304 Wallisellen (CH); Brönnimann, Stefan, 8370 Sirnach (CH); Malacarne, Enrico, 8630 Rüti (CH)
(74) Vertreter: Sutter, Kurt

(56) Entgegenhaltungen:
- CH-A- 432 886
- CH-A5- 683 293
- DE-A1- 3 834 478
- US-A- 5 915 279

## Beschreibung

Die Erfindung betrifft ein Verfahren zur Detektion von Fehlstellen in einem Garn oder Garnvorgänger sowie eine zur Durchführung dieses Verfahrens ausgestaltete Vorrichtung.

Unter "Garnvorgänger" ist dabei eine Faseranordnung zu verstehen, wie sie im Verlauf der Garnherstellung auftritt, z.B. um ein Faservlies nach der Karde oder ein Faserbündel vor dem eigentlichen Spinnprozess.

Bei der Herstellung und Anwendung von Garnen muss eine dauernde Qualitätskontrolle durchgeführt werden. Hierzu sind verschiedene Verfahren bekannt, mit welchen die Qualität des Garns beziehungsweise des Garnvorgängers gemessen werden kann. Solche Verfahren messen beispielsweise die optische Transmission oder Reflektion eines Garns, um daraus Dünnstellen, Dickstellen, Nissen, Fremdfasern, Fehlfarben oder andere in optischer Weise erkennbare Defekte ermitteln zu können. Andere Verfahren verwenden beispielsweise kapazitive Messmethoden.

In US 4 436 472 wird ein Verfahren beschrieben, bei welchem das Messlicht mit einer bestimmten Frequenz moduliert wird, wobei die Modulationsfrequenz von einem Oszillator erzeugt wird, in dessen Rückkopplungskreis die Lichtquelle und der Detektor liegen. Dadurch wird eine sehr frequenzspezifische Messung sichergestellt, was eine Reduktion des Rauschens erlaubt.

WO 99/30108 beschreibt ein Verfahren, welches aus dem gleichen Grunde den Verstärker synchron mit der Modulationsfrequenz der Lichtquelle schaltet. Auch dies erlaubt eine frequenzspezifische Messung mit reduziertem Rauschen. Ein weiteres gattungsgemässes Verfahren wird in CH432886 beschrieben.

Die Aufgabe der vorliegenden Erfindung liegt darin, ein Verfahren und eine Vorrichtung bereitzustellen, welches bzw. welche eine noch bessere Robustheit gegenüber Rauschsignalen besitzen.

Diese Aufgabe wird vom Verfahren beziehungsweise von der Vorrichtung gemäss den unabhängigen Patentansprüchen erfüllt. Demgemäss wird ein Frequenzauswahlschritt durchgeführt, bei dem bei mehreren Frequenzen ermittelt wird, wie gross das Rauschen am Detektor ist. Sodann wird die Messfrequenz in Abhängigkeit der im Frequenzauswahlschritt ermittelten Rauschwerte ausgewählt. Dies erlaubt es, eine Frequenz (oder allenfalls ausgewählte mehrere Frequenzen) zu verwenden, bei welcher (bzw. welchen) ein geringes Rauschen vorhanden ist.

Weitere bevorzugte Ausführungen der Erfindung ergeben sich aus den abhängigen Ansprüchen sowie aus der nun folgenden Beschreibung anhand der Figuren. Dabei zeigen:
Figur 1 eine erste Ausführung der Erfindung,
Figur 2 ein Frequenzspektrum, wie es im Frequenzauswahlschritt ermittelt wird,
Figur 3 eine zweite Ausführung der Erfindung und
Figur 4 eine dritte Ausführung der Erfindung.

In der folgenden Beschreibung sowie in den Patentansprüchen werden folgende Definitionen verwendet:
Unter "Fehlstellen" sind alle detektierbaren Eigenschaften eines Garns zu verstehen, die auf einen Fehler des Garns hinweisen, wie z.B. Dickstellen und Dünnstellen, Nissen, Fremdfasern und andere Fremdstoffe, Fehlfarben etc.
Unter "Rauschen" ist ein nicht konstantes Fehlsignal zu verstehen, welches vom Detektor gemessen wird. Dieses kann z.B. durch mechanische Schwingungen oder elektrische Störsignale erzeugt werden, bei optischen Sensoren auch durch das Umgebungslicht.

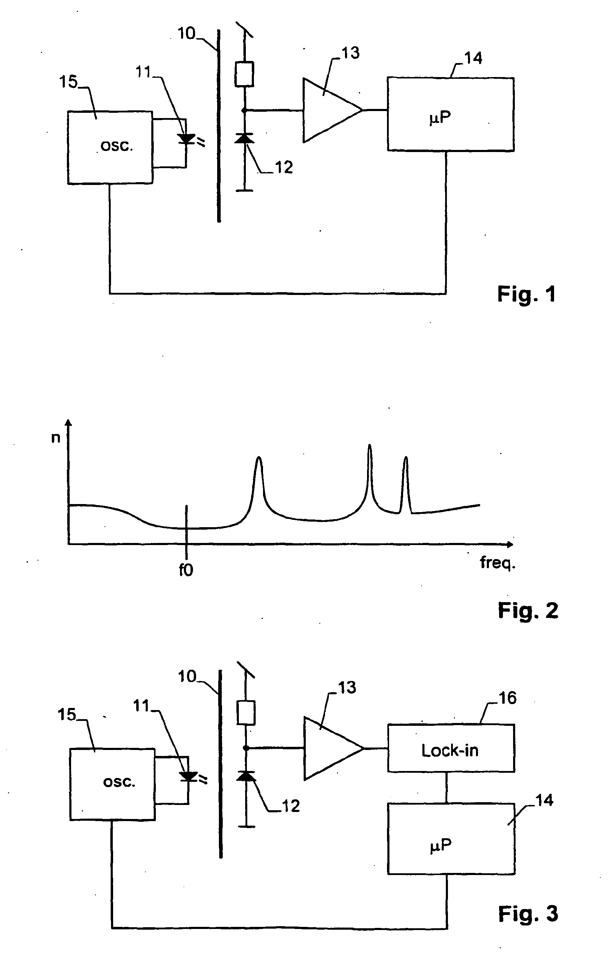

In Figur 1 ist beispielhaft ein optischer Garnsensor dargestellt, bei welchem das Garn 10 zwischen einer Lichtquelle 11 und einem Lichtsensor 12 hindurchgeführt wird. Derartige Anordnungen sind dem Fachmann bekannt. Sie dienen beispielsweise dazu, Dickstellen oder Dünnstellen im Garn zu erfassen, indem vom Sensor 12 ein Signal erzeugt wird, dessen Grösse von der Breite beziehungsweise der Dicke des Garns abhängt. Es ist allerdings zu beachten, dass die Erfindung auch im Zusammenhang mit anderen Messprinzipien angewendet werden kann. Wie bereits erwähnt, können z.B. Reflektionsmessungen oder Kapazitätsmessungen, sowie auch Kombinationen mehrerer Messprinzipien eingesetzt werden. Entsprechende Verfahren sind dem Fachmann bekannt. Die optische Messung kann bei einer oder mehreren Wellenlängen im sichtbaren, infraroten und/oder ultravioletten Spektralbereich durchgeführt werden.

In der vorliegenden Ausführung wird das Signal vom Sensor 12 in einem Verstärker 13 verstärkt und sodann (nach Konversion in ein Digitalsignal) einem Mikroprozessor 14 zugeführt.

Wie aus Figur 1 ersichtlich, wird die Lichtquelle 11 von einem Oszillator 15 angesteuert, der mit einer Frequenz arbeitet, welche vom Mikroprozessor 14 vorgegeben wird. Die Frequenz des Oszillators 15, mit welcher der Strom durch die Lichtquelle 11 moduliert wird, wird im Folgenden die Messfrequenz genannt.

In der vorliegenden Ausführung ist der Mikroprozessor 14 dazu ausgestaltet, vor der eigentlichen Messung einen Frequenzauswahlschritt durchzuführen. Dieser Frequenzauswahlschritt wird ohne Garn 10 oder bei angehaltenem Garn, stationärem Garn oder mit einem Referenzobjekt durchgeführt. Beim Referenzobjekt kann es sich z.B. um ein fehlerfreies Garn handeln. In all diesen Fällen müsste der Sensor 12 bei rauschfreiem Betrieb ein konstantes Signal liefern. Wie eingangs erwähnt, ist das Signal des Sensors 12 jedoch immer mit gewissen Störanteilen, das heisst mit einem Rauschen, belastet. Ziel des Frequenzauswahlschritts ist es, die Natur dieses Rauschens besser kennen zu lernen, so dass die eigentliche spätere Messung in einem Frequenzbereich stattfinden kann, in welchem das Rauschen gering ist.

Hierzu misst der Mikroprozessor 14 in der ersten Ausführung der Erfindung im Frequenzauswahlschritt den zeitlichen Verlauf des Signals vom Detektor und zerlegt dieses in seine Frequenzanteile. Konkret kann dies zum Beispiel geschehen, indem die zu unterschiedlichen Zeiten gemessenen Signalwerte vom Sensor 12 einer FFT- oder DCT-Transformation unterworfen werden. Daraus ergibt sich ein Frequenzspektrum, wie es als qualitatives Beispiel in Figur 2 dargestellt ist. Auf der y-Achse ist dabei die Signalamplitude n bei der jeweiligen Frequenz aufgetragen.

Sodann analysiert der Mikroprozessor 14 das Spektrum und ermittelt einen Bereich mit besonders geringem Rauschen. Im dargestellten Beispiel ist die Frequenz f0.

Nun kann die eigentliche Messung beginnen. Hierzu betreibt der Mikroprozessor 14 den Oszillator 15 bei der Messfrequenz f0, und es wird eine Messung am laufenden Garn 10 durchgeführt. Das Messsignal vom Sensor 12 wird im Mikroprozessor 14 wiederum in seine Frequenzanteile zerlegt und es wird lediglich die Messamplitude bei der Frequenz f0 berücksichtigt.

Eine zweite Ausführung der Erfindung wird in Figur 3 gezeigt. Während in der ersten Ausführung die Frequenzanalyse vom Mikroprozessor 14 durchgeführt wurde, ist in der zweiten Ausführung hierzu ein frequenzselektives Filter 16 vorgesehen, beispielsweise in Form eines so genannten Lock-in-Verstärkers. Die Durchlassfrequenz des frequenzselektiven Filters kann vom Mikroprozessor 14 gewählt werden. Ansonsten ist die zweite Ausführung gleich aufgebaut wie die Erste.

Auch bei der zweiten Ausführung wird zuerst ein Frequenzauswahlschritt durchgeführt, bei welchem das System in einen Zustand gebracht wird, bei welchem das Signal am Ausgang des Verstärkers 13 an sich Null sein sollte. Nun misst der Mikroprozessor 14 das Signal am Ausgang des Filters 16 bei einer Reihe von möglichen Frequenzen. Hierzu werden das Filter 16 und der Oszillator 15 entsprechend durchgestimmt. Dann wählt der Mikroprozessor 14 diejenige Frequenz als Messfrequenz, bei welcher er das geringste Rauschen gemessen hat. Sodann stellt der Mikroprozessor 14 die Frequenz des Filters 16 und des Oszillators 15 auf diese Messfrequenz ein, und es kann eine normale Messung durchgeführt werden.

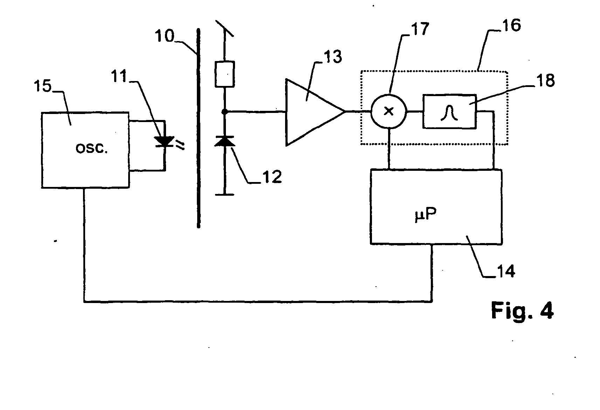

Vorzugsweise wird als Filter 16 ein möglichst schmales Bandpassfilter eingesetzt. Der Lock-in-Verstärker der Ausführung nach Figur 3 ist dafür nur ein Beispiel. Ein weiteres Beispiel wird in der Ausführung nach Figur 4 gezeigt. Hier ist das Filter 16 als so genanntes Zwischenfrequenz-Filter (ZF-Filter) ausgeführt, bei welchem das Eingangssignal in einem Mischer 17 mit einer (vom Mikroprozessor 14 vorgegebenen) Hilfsfrequenz f1 multipliziert und sodann einem schmalbandigen Zwischenfrequenz-Filter 18 zugeführt wird, welches nur Signale mit einer (fest vorgegebenen) Frequenz f2 passieren lässt. Dies erlaubt es dem Mikroprozessor 14, Signale bei der Frequenz f1 - f2 zu messen, wobei diese Frequenz durch Ändern von f1 geändert werden kann.

Ansonsten ist die Ausführung nach Figur 4 gleich aufgebaut wie jene nach Figur 3, und auch der Frequenzauswahlschritt findet in gleicher Weise statt.

In den soweit gezeigten Ausführungen wurde das Garn beziehungsweise der Garnvorgänger mit einem Lichtsignal abgetastet, das mit der Messfrequenz moduliert wurde. Mit anderen Worten wurde an das Garn ein elektromagnetisches Feld angelegt und die Reaktion des Garns auf dieses Feld gemessen. Wie bereits erwähnt, ist jedoch z.B. auch eine kapazitive Messung des Garns möglich. Hierbei wird das Garn durch einen elektrischen Kondensator geführt, wo ein elektrisches Feld an das Garn angelegt wird. Auch in diesem Fall wird die Messung vorzugsweise bei einer Messfrequenz durchgeführt, mit welcher das elektrische Feld im Kondensator moduliert wird. Zur Bestimmung der Messfrequenz kann wiederum der oben erwähnte Frequenzauswahlschritt durchgeführt werden, um diejenige Frequenz zu bestimmen, bei welcher das geringste Rauschen vorhanden ist.

Anstelle einer optischen oder kapazitiven Messung kann beispielsweise auch eine andere elektromagnetische oder berührungslose Messung verwendet werden.

Im Frequenzauswahlschritt kann die Frequenz des optischen beziehungsweise elektrischen Feldes praktisch kontinuierlich durchgestimmt und ein entsprechend detailliertes Rauschspektrum gemessen werden. Denkbar ist jedoch auch, im Frequenzauswahlschritt nur bei einer geringen Zahl von Frequenzen Messungen durchzuführen, was es unter Umständen erlaubt, den schaltungstechnischen Aufwand zu reduzieren.

Vorzugsweise ist ein Detektor mit einem durchstimmbaren Filter 16 vorgesehen, welches im Frequenzauswahlschritt nacheinander auf mehrere Frequenzen abgestimmt werden kann. In der normalen Messung wird sodann das gleiche Filter 16 auf die im Frequenzauswahlschritt bestimmte Messfrequenz eingestellt. In den Ausführungen nach Figur 3 und 4 wurde das Filter 16 in Hardware realisiert, während in der Ausführung nach Figur 1 eine reine Softwarelösung gewählt wurde.

Denkbar ist auch, dass der Detektor mehrere unterschiedliche Sensoren aufweist, welche für jeweils unterschiedliche Frequenzen bzw. Frequenzbereiche empfindlich sind. Im Frequenzauswahlschritt wird bestimmt, welcher der Sensoren das geringste Störsignal zeigt. Dieser wird sodann in der normalen Messung verwendet.

Denkbar ist weiter, in der "normalen" Messung bei mehreren Messfrequenzen zu messen, wobei die bei den einzelnen Messfrequenzen gemessenen Signale gewichtet addiert werden. Die Gewichtung ist abhängig von der Stärke des Rauschens, das bei diesen Frequenzen im Frequenzauswahlschritt ermittelt wurde, z.B. indem sie umgekehrt proportional zur jeweiligen Rauschamplitude gewählt wird.

In den soweit gezeigten Ausführungen besitzt der Detektor einen Lichtsensor 12, mit welchem die Transmission oder Reflektion des Garns beziehungsweise Garnvorgängers gemessen wird. Wie bereits erwähnt, kann jedoch auch eine Kapazitätsmessung durchgeführt werden. Denkbar ist auch die Kombination mehrerer Sensoren, zum Beispiel von Sensoren für unterschiedliche Farben oder von optischen und kapazitiven Sensoren.

Die hier gezeigte Vorrichtung beziehungsweise das beschriebene Verfahren eignen sich besonders für Garnreiniger, sie können jedoch auch in anderen Schritten bei der Herstellung und Verarbeitung von Garnen eingesetzt werden.

## Patentansprüche

1. Verfahren zur Detektion von Fehlstellen in einem Garn oder Garnvorgänger mit einem Detektor (12, 13, 16), bei welchem mindestens ein Parameter des Garns bzw. Garnvorgängers bei mindestens einer Messfrequenz gemessen wird, **dadurch gekennzeichnet, dass** in einem Frequenzauswahlschritt bei mehreren Frequenzen ermittelt wird, wie gross ein Rauschwert am Detektor (12, 13, 16) ist, wobei die Messfrequenz abhängig von den im Frequenzauswahlschritt bestimmten Rauschwerten ist.

2. Verfahren nach Anspruch 1, wobei die Messfrequenz zu derjenigen Frequenz gesetzt wird, die im Frequenzauswahlschritt das geringste Rauschen besitzt.

3. Verfahren nach einem der vorangehenden Ansprüche, wobei im Frequenzauswahlschritt eine Messung ohne Garn bzw. Garnvorgänger, mit stationärem Garn bzw. Garnvorgänger oder mit einem Referenzobjekt durchgeführt wird.

4. Verfahren nach einem der vorangehenden Ansprüche, wobei im Frequenzauswahlschritt ein zeitlicher Verlauf eines Signals vom Detektor (12, 13, 16) ermittelt und dieses Signal in seine Frequenzanteile zerlegt wird.

5. Verfahren nach einem der vorangehenden Ansprüche, wobei das Garn bzw. der Garnvorgänger mit einem Feld abgetastet wird, welches mit der Messfrequenz moduliert ist, und wobei mit dem Detektor (12, 13, 16) eine Reaktion des Garns bzw. Garnvorgängers auf das Feld gemessen wird.

6. Verfahren nach Anspruch 5, wobei im Frequenzauswahlschritt Felder verwendet werden, welche unterschiedliche Frequenzen haben, und wobei für jede Frequenz die Reaktion des Garns bzw. Garnvorgängers auf das Feld gemessen wird.

7. Verfahren nach einem der vorangehenden Ansprüche, wobei der Detektor (12, 13, 16) ein durchstimmbares Filter aufweist, welches im Frequenzauswahlschritt nacheinander auf mehrere Frequenzen abgestimmt und dann auf die Messfrequenz abgestimmt wird.

8. Verfahren nach einem der vorangehenden Ansprüche, wobei der Detektor (12, 13, 16) einen Lichtsensor aufweist, mit welchem eine Transmission oder Reflektion des Garns bzw. Garnvorgängers gemessen wird, wobei das Garn bzw. der Garnvorgänger mit Licht aus einer Lichtquelle (11) beaufschlagt wird, und wobei ein Strom durch die Lichtquelle mit der Messfrequenz moduliert wird,

9. Verfahren nach einem der vorangehenden Ansprüche, wobei bei mehreren Messfrequenzen gemessen wird, wobei die Signale bei den einzelnen Messfrequenzen abhängig vom im Frequenzauswahlschritt bestimmten Rauschen bei den Messfrequenzen gewichtet werden.

10. Vorrichtung zur Detektion von Fehlstellen in einem Garn bzw. Garnvorgänger mit einem Mikroprozessor (14) ausgestaltet zur Durchführung des Verfahrens nach einem der vorangehenden Ansprüche.

## Claims

1. Method for detecting flaws in a yarn or yarn precursor by means of a detector (12, 13, 16), by which at least a parameter of the yarn or yarn precursor respectively is measured at at least a measuring frequency, **characterized in that**, in a frequency selection step, it is determined, at a plurality of frequencies, how high is a noise value at the detector (12, 13, 16), wherein the measuring frequency depends on the noise values determined in the frequency selection step.

2. Method according to claim 1, wherein the measuring frequency is set to that frequency which has the lowest noise in the frequency selection step.

3. Method according to one of the preceding claims, wherein a measurement without yarn or yarn precursor respectively, with a stationary yarn or yarn precursor respectively or with a reference object, is carried out in the frequency selection step.

4. Method according to one of the preceding claims, wherein a time profile of a signal is determined by the detector (12, 13, 16) in the frequency selection step and this signal is decomposed into its frequency components.

5. Method according to one of the preceding claims, wherein the yarn or the yarn precursor, respectively, is scanned by means of a field which is modulated by the measuring frequency, and wherein a reaction of the yarn or yarn precursor respectively to the field is measured with the detector (12, 13, 16).

6. Method according to claim 5, wherein fields having different frequencies are used in the frequency selection step, and wherein the reaction of the yarn or yarn precursor respectively to the field is measured for each frequency.

7. Method according to one of the preceding claims, wherein the detector (12, 13, 16) has a tunable filter which is successively tuned to a plurality of frequencies in the frequency selection step and then tuned to the measuring frequency.

8. Method according to one of the preceding claims, wherein the detector (12, 13, 16) has a light sensor by means of which a transmission or reflection of the yarn or yarn precursor respectively is measured, wherein the yarn or yarn precursor respectively is subjected to light from a light source (11), and wherein a current through the light source is modulated with the measuring frequency.

9. Method according to one of the preceding claims, wherein it is measured at a plurality of frequencies, wherein the signals are weighted at the individual measuring frequencies depending on the noise at the measuring frequencies determined in the frequency selection step.

10. Device for detecting flaws in a yarn or yarn precursor respectively with a microprocessor (14) adapted to carry out the method according to one of the preceding claims.

## Revendications

1. Procédé de détection de défauts dans un fil ou une amorce de fil avec un détecteur (12, 13, 16), dans lequel au moins un paramètre du fil ou de l'amorce de fil est mesuré pour au moins une fréquence de mesure, **caractérisé en ce que** la taille d'une valeur de souffle au niveau du détecteur (12, 13, 16) est déterminée pour plusieurs fréquences dans une étape de sélection de fréquence, la fréquence de mesure étant dépendante des valeurs de souffle déterminées dans l'étape de sélection de fréquence.

2. Procédé selon la revendication 1, dans lequel la fréquence de mesure est ajustée à la fréquence qui possède le souffle le plus faible dans l'étape de sélection de fréquence.

3. Procédé selon l'une quelconque des revendications précédentes, dans lequel une mesure est mise en oeuvre dans l'étape de sélection de fréquence sans fil ou amorce de fil, avec un fil ou une amorce de fil stationnaire, ou avec un objet de référence.

4. Procédé selon l'une quelconque des revendications précédentes, dans lequel une évaluation temporelle d'un signal du détecteur (12, 13, 16) est déterminée dans l'étape de sélection de fréquence et ce signal est décomposé en ses composantes de fréquence.

5. Procédé selon l'une quelconque des revendications précédentes, dans lequel le fil ou l'amorce de fil est exploré(e) avec un champ qui est modulé avec la fréquence de mesure, et dans lequel une réaction du fil ou de l'amorce de fil sur le champ est mesurée par le détecteur (12, 13, 16).

6. Procédé selon la revendication 5, dans lequel des champs ayant des fréquences différentes sont utilisés dans l'étape de sélection de fréquence, et dans lequel la réaction du fil ou de l'amorce de fil sur le champ est mesurée pour chaque fréquence.

7. Procédé selon l'une quelconque des revendications précédentes, dans lequel le détecteur (12, 13, 16) présente un filtre accordable qui est accordé successivement sur plusieurs fréquences dans l'étape de sélection de fréquence et est ensuite accordé à la fréquence de mesure.

8. Procédé selon l'une quelconque des revendications précédentes, dans lequel le détecteur (12, 13, 16) présente un capteur de lumière avec lequel une transmission ou une réflexion du fil ou de l'amorce de fil est mesurée, le fil ou l'amorce de fil étant soumis(e) à de la lumière provenant d'une source lumineuse (11), et un courant étant modulé avec la fréquence de mesure grâce à la source lumineuse.

9. Procédé selon l'une quelconque des revendications précédentes, dans lequel une mesure est effectuée pour plusieurs fréquences de mesures, les signaux étant pondérés pour les fréquences de mesures individuelles en fonction du souffle déterminé dans l'étape de sélection de fréquence pour les fréquences de mesure.

10. Dispositif de détection de défauts dans un fil ou une amorce de fil comprenant un microprocesseur (14) conçu pour la mise en oeuvre du procédé selon l'une quelconque des revendications précédentes.
